# EUROPÄISCHE PATENTSCHRIFT

(11) **EP 1 534 177 B1**
(45) Veröffentlichungstag und Bekanntmachung des Hinweises auf die Patenterteilung: **16.03.2011**
(21) Anmeldenummer: 03793802.4
(22) Anmeldetag: 04.09.2003
(51) Int. Cl.: A61F 2/06

(54) **GEFÄSSPROTHESE, INSBESONDERE ZUM ERSATZ DER AORTA ASCENDENS**
VASCULAR PROSTHESIS, ESPECIALLY FOR REPLACING THE ASCENDING AORTA
PROTHESE VASCULAIRE NOTAMMENT DESTINEE A REMPLACER L'AORTE ASCENDANTE

(30) Priorität: 05.09.2002 DE 10242154
(43) Veröffentlichungstag der Anmeldung: 01.06.2005
(73) Patentinhaber: Sievers, Hans-Hinrich, Prof. Dr. med., 24119 Kronshagen (DE)
(72) Erfinder: Sievers, Hans-Hinrich, Prof. Dr. med., 24119 Kronshagen (DE)
(74) Vertreter: Patentanwälte Ruff, Wilhelm, Beier, Dauster & Partner
(86) Internationale Anmeldenummer: PCT/EP2003/009797
(87) Internationale Veröffentlichungsnummer: WO 2004/021925

(56) Entgegenhaltungen:
- EP-A- 0 955 019
- WO-A-01/52776
- GB-A- 2 312 485
- US-B1- 6 375 679

## Beschreibung

Die vorliegende Erfindung betrifft eine Gefäßprothese, insbesondere zum Ersatz der Aorta ascendens in Form einer geraden Röhre.

Gefäßprothesen, insbesondere zum Ersatz von herznahen Bereichen der Aorta, sind aus dem Stand der Technik bekannt. Verschiedenste Indikationen verlangen den Einsatz solcher Gefäßprothesen, wie beispielsweise Aortenwurzelerweiterungen, Aortendissektion oder Marfan-Syndrom. Beispielsweise beschreiben Tirone E. David und Christopher M. Feindel in "The Journal of Thoracic and Cardiovascular Surgery" (Volume 103, No. 4, April 1992) röhrenförmige Dacron-Gefäßprothesen, die mit Kollagen imprägniert sind. Diese Gefäßprothesen werden nach Entfernung der aneurysmatischen Teilen der Aorta ascendens und der Sinusse unter Erhalt der Aortenklappen und Teilen der Arterienwand eingesetzt. Diese Gefäßprothesen sind den natürlichen anatomischen Gegebenheiten nur schlecht nachempfunden, da ihnen beispielsweise Strukturen fehlen, welche die entfernten Sinusse ersetzen könnten. Sie bestehen lediglich aus einer geraden homogenen Röhre.

Yacoub et al. beschreiben in "The Journal of Thoracic and Cardiovascular Surgery" (Mai 1998, Seite 1080 ff.) eine röhrenförmige Gefäßprothese zum Ersatz der Aorta ascendens, die an ihrem unteren Ende zungenförmige Fortsätze aufweist, welche die entfernten Sinuse ersetzen sollen. Durch diese zungenartigen Fortsätze können die natürlichen anatomischen Gegebenheiten nur bedingt imitiert werden. Zudem ist es relativ aufwendig, eine solche Gefäßprothese mit zungenartigen Fortsätzen einzunähen.

WO 01/52 776 beschreibt eine Gefäßprothese für den Ersatz der Aorta ascendens, die an ihrem unteren Ende (distales Ende) Sinusse zum Ersatz der natürlichen Sinusse aufweist. Diese künstlichen Sinusse sind an das distale Ende der Prothese angenäht. Durch die angenähten Sinusse entstehen am distalen Ende der Gefäßprothese bogenförmige Ausbuchtungen, hervorgerufen durch die Ränder der Sinusse. Solche mit Sinussen versehenen Gefäßprothesen sind schwierig zu implantieren. Weiterhin beschreibt diese Schrift spezielle Gefäßprothesen, welche körperfremde Aortenklappen tierischen Ursprungs aufweisen, wobei diese Aortenklappen an die bogenförmigen Ränder der Sinusse angenäht werden.

In der 5. Ausgabe von "Surgical sense" wird eine Gefäßprothese zum Ersatz der Aorta ascendens beschrieben, welche eine umlaufende bauchförmige Erweiterung aufweist, die die natürlichen Sinuse ersetzen soll. Ähnliches wird auch in EP 0 955 019 beschrieben. Auch diese Versuche, die natürlichen Verhältnisse in der Aorta ascendens zu imitieren, sind nur bedingt dazu geeignet. Es wird hier in keinster Weise der Tatsache Rechnung getragen, dass die natürliche Aorta getrennte Sinusse aufweist.

US-B-6375679 weist eine Gefäßprothese mit den Merkmalen des Oberbegriffs aus Anspruch 1 auf.

Aufgabe der vorliegenden Erfindung ist es, eine Gefäßprothese zu schaffen, die die Nachteile des Standes der Technik überwindet. Sie soll insbesondere die natürlichen anatomischen Gegebenheiten möglichst optimal wiedergeben sowie vom Operateur in einfacher Weise zu implantieren sein.

Diese Aufgabe wird erfindungsgemäß durch eine Gefäßprothese der eingangs genannten Art gelöst, die dadurch gekennzeichnet ist, dass die Sinusse über ihren gesamten Umfang mit Prothesenwandmaterial verbunden sind, und das untere Ende der Röhre im wesentlichen geradlinig abgeschlossen ist. Somit endet das untere Ende der Prothese im wesentlichen gerade und ist an einem geraden Stumpf der Aortenwurzel des Herzens annähbar. Streben der Aortenwand sind an der Innenseite der Röhre im Bereich zwischen den Sinusen fixierbar. Besonders bevorzugt weist die Röhre im herznahen Berich mindestens zwei, insbesondere drei Aussparungen, in die jeweils ein Sinus direkt eingesetzt ist, auf. Dadurch wird der Normalfall der natürlichen anatomischen Gegebenheiten optimal imitiert. Ein wesentlicher Vorzug der erfindungsgemäßen Lösung ist darin zu sehen, dass die Sinusse bereits bei der Herstellung der Prothese dicht mit den sie umgebenden Prothesenwandteilen verbunden, insbesondere vernäht werden können, so dass diese für die Funktion der Prothese wichtige Arbeit nicht dem Operateur zugemutet werden muss.

Durch das Vorhandensein der Sinusse wird eine physiologische Klappenbewegung gewährleistet, was eine längere "Haltbarkeit" der patienteneigenen Klappensegel mit sich bringt. Durch die Tatsache, dass das untere Ende der Röhre in der erfindungsgemäßen Gefäßprothese im wesentlichen geradlinig abgeschlossen ist, wird eine erleichterte Implantation für den Operateur erreicht. Des weiteren ist dadurch eine sichere basale Verankerung der Prothese sowie eine sichere Blutstillung bzw. Vermeidung von Blutungen möglich. Nicht zuletzt wird durch die erleichterte Einbauweise eine kürzere OP-Zeit erreicht. Mit Vorteil schließt sich am herznahen unteren Rand der Prothese an die Sinusse ein rohrförmiger textiler Prothesenwandabschnitt an. Dadurch wird ebenfalls ein erleichtertes Implantieren erzielt.

Im Vergleich zu manch anderen Gefäßprothesen aus dem Stand der Technik werden bei der erfindungsgemäßen Gefäßprothese die patienteneigenen Aortenklappensegel erhalten. Es werden die Aortenwurzel und Teile der Aorta ascendens resiziert und durch die Gefäßprothese ersetzt. Hierbei werden auch die klappennahen Aortenausbuchtungen (Sinus Valsalvae) vollständig entfernt.

Es ist bevorzugt, dass die erfindungsgemäße Gefäßprothese eine poröse Wandung aufweist, da dies das Einwachsen von Gewebe und kleinen Blutgefäßen fördert. Besonders bevorzugt ist es, dass die Gefäßprothese aus textilem Material (z.B. Dacron) besteht und insbesondere gewirkt oder gewebt ist. Auch ist es bevorzugt, dass die Gefäßprothese, insbesondere auf der Außenwandung, mit einem natürlichen oder synthetischen resorbierbaren Material beschichtet ist. Hierfür eignet sich beispielsweise Gelatine, insbesondere vernetzte Gelatine. Dieses Material wird nach der Implantation langsam resorbiert und durch körpereigenes Gewebe ersetzt.

Besonders bevorzugt ist es, dass die Röhre einen im wesentlichen geradlinigen Verlauf aufweist. Dies entspricht auch den anatomischen Gegebenheiten der Aorta im herznahen Bereich (Aorta ascendens).

An die geradlinige Prothese kann am vom Herz abweisenden Ende die Aorta ascendens des Patienten oder eine die Aorta ascendens und den Aortenbogen bildende gesonderte Prothese anschließen. Eine die Aorta ascendens und den anschließenden Aortenbogen bildende Prothese kann auch Bestandteil der erfindungsgemäßen Prothese sein und kann dann z. B. in einem vordefinierten Bereich von dieser vor der Implantation abgetrennt und nach dem Einnähen der erfindungsgemäßen Prothese wieder mit dieser verbunden werden.

Mit Vorteil befinden sich die Ausparungen in einem Abstand zum unteren Prothesenende. Dieser Abstand beträgt vorzugsweise ca. 0,5 cm bis ca. 6 cm, insbesondere ca. 1 bis 3 cm. Durch diesen Abstand werden ebenfalls die natürlichen anatomischen Verhältnisse wiedergegeben sowie ein erleichtertes Einnähen durch den Operateur ermöglicht. Auch ist es möglich, dass der Abstand absichtlich etwas größer gehalten wird (z.B. über 6 cm) und der gewünschte Abstand durch Abschneiden eines bestimmten Teils der Röhre erreicht wird. Die Prothese, insbesondere ihr herznahes Ende, ist frei von Herzklappen.

Bei einer besonders bevorzugten Ausführungsform der erfindungsgemäßen Gefäßprothese sind zwischen den Sinussen freie, längs verlaufende Stegabschnitte der Röhre vorhanden. Beim Entfernen der erkrankten Aortenabschnitte werden nämlich die natürlichen Sinusse entfernt, indem man entlang ihren Außenrändern schneidet. Dabei bleiben ca. 8 mm breite Streben der Aorta ascendens zurück. Diese Streben können nun an den Innenseiten der vorhandenen freien, längsverlaufenden Stegabschnitten der Röhre fixiert werden. Die Streben können an den Stegabschnitten beispielsweise angenäht oder angeklebt werden.

Die erfindungsgemäße Gefäßprothese weist eine Plissierung mit Falten auf.

Die Plissierung kann wendelförmig oder als geschlossene Ringe ausgebildet sein. Allgemein bewirkt eine Plissierung eine Elastizität der Gefäßprothese. Die Röhre kann ca. 2 bis 8, vorzugsweise ca. 3 bis 5 Falten pro cm Prothesenlänge aufweisen.

Üblicherweise weist die erfindungsgemäße Prothese einen Durchmesser von 24-38 mm auf. Die Sinusse können aus einem anderen Material als die Röhre bestehen. Sie können beispielsweise auch aus einem nicht textilen Material bestehen. Bevorzugt bestehen die Sinusse jedoch aus textilem Material, insbesondere aus dem selben Material wie die Röhre besteht. Die Sinusse können verschiedene Formen aufweisen. Besonders bevorzugt sind sie schildförmig. Die erfindungsgemäße Gefäßprothese weist Sinusse mit einer Falten aufweisenden Plissierung auf. Die Falten der Plissierung können in Umfangsrichtung der Prothese verlaufen. Vorzugsweise verlaufen die Falten der Plissierung der Sinusse jedoch in Längsrichtung zur Prothese. Vorzugsweise weisen die Sinusse ca. 3 bis 12, vorzugsweise ca. 5 bis 10, insbesondere 6-8, Falten pro cm auf.

Besonders bevorzugt weisen die Sinusse eine größere Fläche auf als die Aussparungen. Zum einen können dadurch die Sinusse dichter und in einfacher Weise in die Aussparungen eingesetzt werden. Zum anderen entstehen dadurch im Bereich der Sinusse Ausbuchtungen, was ja auch der Anatomie der natürlichen Sinusse entspricht. Je größer die Fläche der Sinusse ist, desto stärker ist auch die Ausbuchtung.

Bei einer besonders bevorzugten Ausführungsform der erfindungsgemäßen Gefäßprothese sind die Sinusse mit dem Rand der Aussparungen dicht vernäht. Hierbei kommen unterschiedliche Nähte in Frage (z.B. Steppstich, "Safety-Naht").

Bei einer weiteren Ausführungsform der erfindungsgemäßen Gefäßprothese sind die Sinusse mit dem Rand der Aussparungen verklebt.

Mit 'Vorteil weist die Röhre drei auf gleicher Höhe liegende axialsymmetrische Aussparungen auf, in die jeweils ein Sinus eingesetzt ist, wobei insbesondere die Breite der Sinusse an ihrer breitesten Stelle ca. 1/3 Umfang minus 8 mm beträgt.

### Beispiel:

Durchmesser ist 30 mm: U = II x 30 ≅ 94 mm; 1/3 U minus 8 mm ≅ 23 mm.

Die Aussparungen können unterschiedliche Formen aufweisen. Vorzugsweise sind die Aussparungen schildförmig bzw. wappenförmig.

Bevorzugt ist die erfindungsgemäße Gefäßprothese vorgefertigt.

### Herstellungsbeispiele für eine erfindungsgemäße Gefäßprothese

Eine unplissierte, gerade Röhre mit einem Durchmesser von 30 mm wird auf einen passenden geraden Stab aufgezogen und thermisch fixiert. Mit Hilfe einer Schablone (Sinusbreite 23 mm, Sinushöhe 18 mm) werden aus einem herznahen Abschnitt der geraden Röhre drei schildförmige Öffnungen ausgeschnitten, so dass zwischen ihnen jeweils ein Steg von 8 mm Breite erhalten bleibt.

Aus einer plissierten Prothese mit einem Durchmesser von 30 mm werden nun ebenfalls mittels einer Schablone drei schildförmige Sinusstücke herausgeschnitten, die in Höhe und Breite jeweils 3 mm größer sind als die oben beschriebenen Öffnungen. Die Schildoberkante liegt hierbei parallel zu den Plissierungen. Um ein Ausfransen zu vermeiden, können die jeweiligen Schnittkanten mit einem heißen Draht verschweißt (gekautert) oder verklebt werden. Diese Stücke können auch mit Knopflochstichen umrandet werden. Diese vorbehandelten Sinusse werden auf dem Abschnitt der Prothese-festgenäht; dabei werden ihre Plissierfalten etwas zusammengedrückt. Bei einer maschinellen Naht kommen zum dichten Annähen der Sinusstücke zum Beispiel ein Steppstich oder eine sogenannte "Safety-Naht" in Frage.

Unterschiedliche Ausbuchtungen der Sinuse können dadurch erzielt werden, indem verschieden breite Prothesenstücke auf die Öffnungen appliziert werden.

Neben dem beschriebenen Herstellungsverfahren der erfindungsgemäßen Gefäßprothese (Ausschneiden von Aussparungen und Applizieren der Sinusse) sind auch noch andere Herstellungsverfahren denkbar:
Es kann beispielsweise auch zuerst die Ursprungsprothese mit drei Sinuszungen vorgefertigt werden und dann anschließend ein Endabschnitt mit den Zwischenstegen angebracht werden.

Auch ein primäres Einweben, Stricken etc. der Sinusse in die Röhre der Prothese ist denkbar.

Auch eine plastische Verformung der Ursprungsprothese und Ausformung der Sinusse ist denkbar.

Weitere Einzelheiten und Merkmale der Erfindung ergeben sich aus der nachfolgenden Beschreibung einer bevorzugten Ausführungsform in Verbindung mit den Unteransprüchen. Hierbei können die jeweiligen Merkmale für sich allein oder zu mehreren in Kombination miteinander verwirklicht sein.

### Figurenbeschreibung

In den Zeichnungen zeigen:

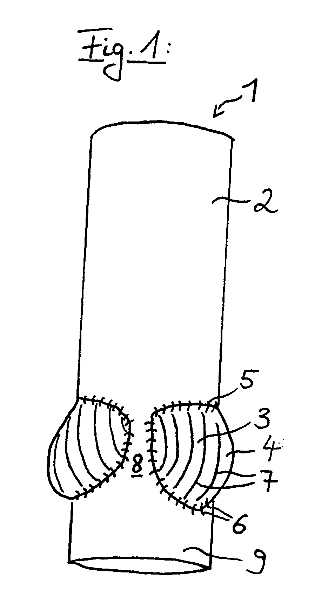

- Figur 1:: Seitenansicht einer erfindungsgemäßen Gefäßprothese aus textilem Material mit eingenähten Sinussen.

Die Gefäßprothese 1 ist geeignet zum Ersatz von Teilen der Aorta, insbesondere zum Ersatz der Aorta ascendens inklusive den Sinussen. Die Gefäßprothese 1 weist eine Röhre 2 aus gewebtem multifilem Garn auf. Diese Röhre 2 zeigt einen im wesentlichen geraden Verlauf und ist nicht plissiert. Die Röhre 2 hat einen Durchmesser von z. B. 30 mm. Des weiteren weist die Röhre 2 im herznahen Bereich auf gleicher Höhe drei durch Ausschneiden gebildete Aussparungen 3 auf, welche von den Sinussen 4 überdeckt sind. Die Sinusse 4 sind mit den Rändern der Aussparungen 3 ringsum dicht vernäht. Die Naht 5 besteht im vorliegenden Ausführungsbeispiel aus Knopflochstichen 6. Die Sinusse 4 weisen eine größere Fläche (ca. 3 cm breit und ca. 2,8 cm hoch) auf als die Aussparungen 3 (ca. 2,6 cm breit und ca. 2,5 cm hoch). Dadurch überlappen die Sinusse die Ränder der Aussparungen und es werden die Sinusse zu Ausbuchtungen in der Prothese. So können sie einfach in die Aussparungen eingenäht werden. Des weiteren weisen die Sinusse 4 eine Falten aufweisende Plissierung 7 auf. Diese Falten aufweisende Plissierung der Sinusse 4 verläuft in Längsrichtung der Prothese. Die Sinusse weisen wie auch die Aussparungen 3 eine Schild- bzw. Wappenform auf. Der obere Rand der Sinusse ist geringfügig konvex. Die Seitenränder convergieren leicht nach unten. Der untere Rand ist deutlich konvex. Die Sinusse sind aus dem selben textilen Material wie die Röhre 2. Die Sinusse weisen ca. 7 Falten pro cm auf. Die Sinusse 4 sind in einem Abstand von ca. 4 mm zueinander angeordnet, so dass zwischen den Sinussen ein Stegbereich 8 existiert, der aus Röhrenmaterial besteht. Dieser Stegbereich 8 dient bei der Implantation der Gefäßprothese zur Fixierung der körpereigenen Aortenstege, die beim Ausschneiden der Sinusse der patienteneigenen Aorta ascendens verbleiben. Die Sinusse 4 reichen nicht bis zum unteren Ende der Gefäßprothese. Den letzten herznahen Endabschnitt der Gefäßprothese bildet ein Stück 9 der Röhre 2, welches im wesentlichen geradlinig endet, so dass das untere Ende der Prothese während der Operation in einfacher Weise an der Aortenwurzel des Herzens annähbar ist. Das Stück 9 weist eine Länge von ca. 2 cm auf.

## Patentansprüche

1. Gefäßprothese (1), insbesondere zum Ersatz der Aorta ascendens, in Form einer Röhre (2), wobei die Röhre (2) im herznahen Bereich mindestens zwei Sinusse (4) aufweist, die eine größere Ausdehnung nach außen besitzen als die Röhre (2), wobei die Sinusse (4) über ihren gesamten Umfang mit Prothesenwandmaterial verbunden sind, und das untere Ende (9) der Röhre (2) im wesentlichen geradlinig abgeschlossen ist, sodass das untere Ende der Prothese (1) an der Aortenwurzel des Herzens annähbar und Streben der Aortenwand an der Innenseite der Röhre (2) im Bereich (8) zwischen den Sinussen (4) fixierbar sind, **dadurch gekennzeichnet, dass** die Prothese (1) frei von Herzklappen ist und die Sinusse (4) eine Falten aufweisende Plissierung (7) aufweisen.

2. Gefäßprothese (1) nach Anspruch 1, **dadurch gekennzeichnet, dass** sich am herznahen unteren Rand der Prothese (1) an die Sinusse (4) ein rohrförmiger textiler Prothesenwandabschnitt anschließt.

3. Gefäßprothese (1) nach einem der Ansprüche 1 oder 2, **dadurch gekennzeichnet, dass** die Röhre (2), insbesondere auch die Sinusse (4), aus textilem Material besteht, insbesondere gewirkt oder gewebt ist.

4. Gefäßprothese (1) nach einem der vorhergehenden Ansprüche, **dadurch gekennzeichnet, dass** die Röhre (2) einen im wesentlichen geradlinigen Verlauf aufweist.

5. Gefäßprothese (1) nach einem der vorhergehenden Ansprüche, **dadurch gekennzeichnet, dass** die Röhre (2) im herznahen Bereich mindestens zwei Aussparungen (3) aufweist, in die jeweils ein Sinus (4) dicht eingesetzt ist.

6. Gefäßprothese (1) nach Anspruch 5, **dadurch gekennzeichnet, dass** sich die Aussparungen (3) in einem Abstand zum unteren Prothesenende befinden.

7. Gefäßprothese (1) nach einem der vorhergehenden Ansprüche, **dadurch gekennzeichnet, dass** zwischen den Sinussen (4) freie längsverlaufende Stegabschnitte (8) der Röhre (2) vorhanden sind.

8. Gefäßprothese (1) nach einem der vorhergehenden Ansprüche, **dadurch gekennzeichnet, dass** die Röhre (2) frei von einer Plissierung ist.

9. Gefäßprothese (1) nach einem der vorhergehenden Ansprüche, **dadurch gekennzeichnet, dass** die Röhre (2) einen Durchmesser im Bereich von 24-38mm besitzt.

10. Gefäßprothese (1) nach einem der vorhergehenden Ansprüche, **dadurch gekennzeichnet, dass** die Sinusse (4) aus textilem Material, insbesondere aus dem selben Material wie die Röhre (2), bestehen.

11. Gefäßprothese (1) nach Anspruch 1, **dadurch gekennzeichnet, dass** die Plissierung der Sinusse (4) stärker ausgeprägt ist als die der Röhre (2).

12. Gefäßprothese (1) nach Anspruch 1 oder 11, **dadurch gekennzeichnet, dass** die Sinusse (4) ca. 3 bis 12, vorzugsweise ca. 5 bis 10, insbesondere 6-8, Falten pro cm aufweisen.

13. Gefäßprothese (1) nach einem der Ansprüche 1 bis 9 oder 11 bis 12, **dadurch gekennzeichnet, dass** die Sinusse (4) aus einem anderen Material als die Röhre (2), insbesondere aus einem nichttextilen Material, bestehen.

14. Gefäßprothese (1) nach einem Ansprüche 5 bis 13, **dadurch gekennzeichnet, dass** die Sinusse (4) eine größere Fläche aufweisen als die Aussparungen (3).

15. Gefäßprothese (1) nach einem der Ansprüche 5 bis 14, **dadurch gekennzeichnet, dass** die Sinusse (4) mit dem Rand der Aussparungen (3) vernäht sind.

16. Gefäßprothese (1) nach einem der Ansprüche 5 bis 14, **dadurch gekennzeichnet, dass** die Sinusse (4) mit dem Rand der Aussparungen (3) verklebt sind.

17. Gefäßprothese (1) nach einem der Ansprüche 5 bis 16, **dadurch gekennzeichnet, dass** die Röhre (2) drei axialsymmetrische Aussparungen (3) aufweist in die drei Sinusse (4) eingesetzt sind, wobei insbesondere die Breite der Sinusse (4) an ihrer breitesten Stelle ca. 1/3 Umfang - 8 mm beträgt.

18. Gefäßprothese (1) nach einem der vorhergehenden Ansprüche, **dadurch gekennzeichnet, dass** sie vorgefertigt ist.

## Claims

1. Vascular prosthesis (1), especially for replacing the ascending Aorta, in the form of a tube (2), the tube (2) comprising at least two sinuses (4) located in the region close to the heart, said sinuses expanding further outwards than the tube (2), said sinuses (4) being connected to the prosthesis wall material over their entire circumference, and the lower end (9) of the tube (2) ending essentially in a straight line in such a way that the lower end of the prosthesis (1) is to be sutured to the aortic root of the heart and struts of the aorta wall are to be fixed at the inside of the tube (2) in the region (8) between the sinuses (4), **characterized in that** the prosthesis (1) is free of cardiac valves and the sinuses (4) have a pleating (7) including creases.

2. Vascular prosthesis (1) according to claim 1, **characterized in that** a tubular textile prosthesis wall section follows up to the sinuses (4) at the lower edge of the prosthesis (1) close to the heart.

3. Vascular prosthesis (1) according to claim 1 or 2, **characterized in that** the tube (2), in particular also the sinuses (4), is (are) composed of textile material, in particular is (are) knitted or woven.

4. Vascular prosthesis (1) according to any of the preceding claims, **characterized in that** the tube (2) has an essentially straight extension.

5. Vascular prosthesis (1) according to any of the preceding claims, **characterized in that** the tube (2) has at least two recesses (3) in the region close to the heart, where in each recess one sinus (4) is sealingly inserted.

6. Vascular prosthesis (1) according to claim 5, **characterized in that** the recesses (3) are spaced from the lower end of the prosthesis.

7. Vascular prosthesis (1) according to any of the preceding claims, **characterized in that** between the sinuses (4) free longitudinal web portions (8) of the tube (2) are disposed.

8. Vascular prosthesis (1) according to any of the preceding claims, **characterized in that** the tube (2) is free of pleating.

9. Vascular prosthesis (1) according to any of the preceding claims, **characterized in that** the tube (2) has a diameter in the range from 24 to 38 mm.

10. Vascular prosthesis (1) according to any of the preceding claims, **characterized in that** the sinuses (4) are composed of textile material, in particular of the same material as the tube (2).

11. Vascular prosthesis (1) according to any of the preceding claims, **characterized in that** the pleating of the sinuses (4) is more pronounced than that of the tube (2).

12. Vascular prosthesis (1) according to any of the claims 1 to 11, **characterized in that** the sinuses (4) have about 3 to 12, preferably about 5 to 10, in particular 6 to 8 creases per centimeter (cm).

13. Vascular prosthesis (1) according to any of the claims 1 to 9 or 11 to 12, **characterized in that** the sinuses (4) are composed of a material differing from the material of the tube (2), in particular are composed of a non-textile material.

14. Vascular prosthesis (1) according to any of the claims 5 to 13, **characterized in that** the sinuses (4) have a larger surface area than the recesses (3).

15. Vascular prosthesis (1) according to any of the claims 5 to 14, **characterized in that** the sinuses (4) are sutured to the edge of the recesses (3).

16. Vascular prosthesis (1) according to any of the claims 5 to 14, **characterized in that** the sinuses (4) are adhesively bonded to the edge of the recesses (3).

17. Vascular prosthesis (1) according to any of the claims 5 to 16, **characterized in that** the tube (2) has three axially symmetrical recesses (3) where three sinuses (4) are inserted, in particular the width of the sinuses (4) at the widest point being about 1/3 of the circumference minus 8 mm.

18. Vascular prosthesis (1) according to any of the preceding claims, **characterized in that** it is prefabricated.

## Revendications

1. Prothèse vasculaire (1) destinée en particulier à remplacer l'aorta ascendens et qui présente la forme d'un tube (2),
le tube (2) présentant dans sa partie proche du coeur au moins deux sinus (4) qui s'étendent vers l'extérieur davantage que le tube (2),
les sinus (4) étant reliés sur toute leur périphérie à un matériau de paroi de prothèse,
l'extrémité inférieure (9) du tube (2) étant fermée de manière essentiellement rectiligne de telle sorte que l'extrémité inférieure de la prothèse (1) puisse être suturée à la racine aortique du coeur et que des entretoises de la paroi aortique puissent être fixées sur le côté intérieur du tube (2) dans la zone (8) située entre les sinus (4),
**caractérisée en ce que**
la prothèse (1) ne présente pas de valve cardiaque et **en ce que** les sinus (4) présentent un plissage (7) doté de plis.

2. Prothèse vasculaire (1) selon la revendication 1, **caractérisée en ce qu'**une partie textile des parois de prothèse de forme tubulaire se raccorde aux sinus (4) sur le bord inférieur, proche du coeur, de la prothèse (1).

3. Prothèse vasculaire (1) selon l'une des revendications 1 ou 2, **caractérisée en ce que** le tube (2) et en particulier également les sinus (4) sont constitués d'un matériau textile, en particulier tricoté ou tissé.

4. Prothèse vasculaire (1) selon l'une des revendications précédentes, **caractérisée en ce que** le tube (2) s'étend essentiellement en ligne droite.

5. Prothèse vasculaire (1) selon l'une des revendications précédentes, **caractérisée en ce que** le tube (2) présente dans sa partie proche du coeur au moins deux découpes (3) dans chacune desquelles un sinus (4) est inséré de manière étanche.

6. Prothèse vasculaire (1) selon la revendication 5, **caractérisée en ce que** les découpes (3) sont situées à distance de l'extrémité inférieure de la prothèse.

7. Prothèse vasculaire (1) selon l'une des revendications précédentes, **caractérisée en ce que** des parties libres (8) d'entretoise, s'étendant en longueur, du tube (2) sont prévues entre les sinus (4).

8. Prothèse vasculaire (1) selon l'une des revendications précédentes, **caractérisée en ce que** le tube (2) ne présente pas de plissage.

9. Prothèse vasculaire (1) selon l'une des revendications précédentes, **caractérisée en ce que** le tube (2) possède un diamètre compris dans la plage de 24 à 38 mm.

10. Prothèse vasculaire (1) selon l'une des revendications précédentes, **caractérisée en ce que** les sinus (4) sont constitués d'un matériau textile et en particulier du même matériau que le tube (2).

11. Prothèse vasculaire (1) selon la revendication 1, **caractérisée en ce que** le plissage des sinus (4) est plus marqué que celui du tube (2).

12. Prothèse vasculaire (1) selon la revendication 1 ou 11, **caractérisée en ce que** les sinus (4) présentent environ 3 à 12, de préférence environ 5 à 10 et en particulier 6 à 8 plis par cm.

13. Prothèse vasculaire (1) selon l'une des revendications 1 à 9 ou 11 et 12, **caractérisée en ce que** les sinus (4) sont constitués d'un autre matériau que le tube (2) et en particulier d'un matériau non textile.

14. Prothèse vasculaire (1) selon l'une des revendications 5 à 13, **caractérisée en ce que** la superficie des sinus (4) est plus grande que celle des découpes (3).

15. Prothèse vasculaire (1) selon l'une des revendications 5 à 14, **caractérisée en ce que** les sinus (4) sont cousus sur le bord des découpes (3).

16. Prothèse vasculaire (1) selon l'une des revendications 5 à 14, **caractérisée en ce que** les sinus (4) sont collés sur le bord des découpes (3).

17. Prothèse vasculaire (1) selon l'une des revendications 5 à 16, **caractérisée en ce que** le tube (2) présente trois découpes (3) à symétrie axiale dans lesquelles trois sinus (4) sont insérés, la largeur des sinus (4) étant en particulier d'environ 1/3 de la périphérie - 8 mm en leur emplacement le plus large.

18. Prothèse vasculaire (1) selon l'une des revendications précédentes, **caractérisée en ce qu'**elle est préfabriquée.
